# EUROPÄISCHE PATENTANMELDUNG

(11) **EP 2 446 909 A2**
(43) Veröffentlichungstag der Anmeldung: **02.05.2012**
(21) Anmeldenummer: 11185714.0
(22) Anmeldetag: 19.10.2011
(51) Int. Cl.: A61M 1/36, A61M 5/168, A61M 39/10

(54) **Sicherungseinrichtung zur Kontrolle eines Gefäßzugangs**

(30) Priorität: 26.10.2010 DE 102010049723
(71) Anmelder: Ehni, Bernhard, 73033 Göppingen (DE)
(72) Erfinder: Ehni, Bernhard, 73033 Göppingen (DE)
(74) Vertreter: Ruckh, Rainer Gerhard

(57) **Zusammenfassung**

Die Erfindung betrifft eine Sicherungseinrichtung (1) zur Kontrolle eines Gefäßzugangs eines Lebewesens. Erfindungsgemäß ist ein Sensor vorgesehen, welcher in oder an einem den Gefäßzugang umgebenden Gehäuse (4) angeordnet ist. Mit dem Sensor wird eine in das Gehäuse (4) von dem Gefäßzugang aus einfließende Flüssigkeit, insbesondere Blut, detektiert.

## Beschreibung

Die Erfindung betrifft eine Sicherungseinrichtung gemäß dem Oberbegriff des Anspruchs 1.

Bei spezifischen Behandlungen von insbesondere menschlichen Patienten ist es erforderlich, diesen kontrolliert Blut oder gegebenenfalls andere Flüssigkeiten in eine Blutbahn einzuführen oder auch Blut aus einer Blutbahn zu entnehmen.

Ein Beispiel hierfür ist die Durchführung einer Dialyse. Zur Durchführung einer Blutwäsche, das heißt eines Blutaustauschs, wird dem Patienten eine erste Kanüle in eine Arterie eingeführt, um dem Patienten Blut zur Reinigung zu entnehmen. Weiterhin wird dem Patienten in eine Vene eine zweite Kanüle eingeführt, um gereinigtes Blut wieder dem Patienten zuzuführen. Alternativ kann anstelle einer Kanüle auch ein Injektionskörper in Form eines Schlauchs mittels eines Katheders in die jeweilige Blutbahn eingeführt werden.

Zur Durchführung dieses Prozesses nimmt der Patient auf einer Liege Platz, dann werden die Kanülen in die Arterie und Vene eingeführt. Die Kanülen mit daran anschließenden Blut-führenden Schläuchen werden mit Klebebändern und dergleichen am Patienten fixiert, um ein Herauslösen der Kanülen zu verhindern.

Diese Sicherung der Kanülen ist keineswegs sicher. Bereits durch kleine Bewegungen des Patienten kann sich eine Kanüle aus dem Gefäßzugang lösen. Diese Gefahr ist relativ groß und nicht vernachlässigbar, da sich der Dialysevorgang über einen langen Zeitraum hinzieht. Ein Herauslösen der Kanüle könnte vom Krankenpersonal sofort behoben werden, wenn dieses beim Patienten verbleibt und die Überwachung übernimmt. Da der Dialysevorgang jedoch zeitaufwändig ist, ist der Patient über längere Zeiträume unbeaufsichtigt. Die größte Gefahr für den Patienten besteht darin, wenn sich die in die Vene eingeführte Kanüle aus dem Gefäßzugang löst. Dann gelangt das zurückzuführende Blut nicht mehr in die Blutbahn des Patienten, was zu einem sehr hohen Blutverlust in kurzer Zeit führt. Ist dann das Krankenpersonal nicht sofort zur Stelle, besteht für den Patienten akute Gefahr, dass er verblutet.

Der Erfindung liegt die Aufgabe zugrunde, eine Sicherungseinrichtung bereitzustellen, mittels derer derartige Gefahrensituationen vermieden werden können.

Zur Lösung dieser Aufgabe sind die Merkmale des Anspruchs 1 vorgesehen. Vorteilhafte Ausführungsformen und zweckmäßige Weiterbildungen sind in den Unteransprüchen beschrieben.

Die erfindungsmäße Sicherungseinrichtung dient zur Kontrolle eines Gefäßzugangs eines Lebewesens. Erfindungsgemäß ist ein Sensor vorgesehen, welcher in oder an einem den Gefäßzugang umgebenden Gehäuse angeordnet ist. Mit dem Sensor wird eine in das Gehäuse von dem Gefäßzugang aus einfließende Flüssigkeit, insbesondere Blut, detektiert.

Mit der erfindungsgemäßen Sicherungseinrichtung kann für verschiedenartige Gefäßzugänge das Austreten unterschiedlicher Flüssigkeiten überwacht werden. Beispielsweise können mit der Sicherungseinrichtung Gefäßzugänge zu Lymphgefäßen überwacht werden. Ohne Beschränkung der Allgemeinheit wird im Folgenden auf einen Gefäßzugang an einer Blutbahn Bezug genommen, wobei insbesondere mit der Sicherungseinrichtung ein Herauslösen einer Kanüle oder eines Katheders aus einer Blutbahn oder allgemein eine Leckage kontrolliert wird.

Der Gefäßzugang an einer Blutbahn kann von einem Schlauch, der mit einem Katheter in die Blutbahn eingeführt wird, gebildet sein. Ohne Beschränkung der Allgemeinheit wird im Folgenden auf einen Gefäßzugang, der mittels einer Kanüle hergestellt wird, Bezug genommen. Bei der erfindungsgemäßen Sicherungseinrichtung wird der Umstand ausgenützt, dass bei einem ungewollten Herauslösen einer Kanüle aus dem Gefäßzugang eines Patienten beziehungsweise allgemein bei einer vorhandenen Leckage sofort Blut aus dem Gefäßzugang austritt. Dieses Blut füllt das Gehäuse sofort auf, wobei vorteilhaft die Abmessungen des Gehäuses so dimensioniert sind, dass bereits nach einer sehr kurzen Zeit, vorzugsweise im Sub-Sekundenbereich, eine zumindest partielle Füllung des Gehäuses mit Blut auftritt, die dann vom Sensor sicher detektiert werden kann. Die Sicherungseinrichtung generiert dann sofort ein Alarmsignal, so dass dem Patienten sofort Hilfe geleistet werden kann. Damit können Gefährdungen sicher ausgeschlossen werden.

Die Sicherungseinrichtung bildet vorzugsweise mit der Kanüle und den daran angeschlossenen Schläuchen eine Funktions- und Baueinheit derart, dass bei der Einführung der Kanüle in den Gefäßzugang die Sicherungseinrichtung selbsttätig oder mit wenigen Handgriffen in ihrer Sollposition liegt, so dass das Gehäuse der Sicherungseinrichtung mit dem Sensor auf der Haut des Patienten sicher und relativ dicht aufsitzt, wobei der Gefäßzugang in einem vom Gehäuse und der Haut des Patienten begrenzten Hohlraum angeordnet ist.

Im Notfall, das heißt bei ungewolltem Herauslösen der Kanüle aus dem Gefäßzugang füllt sich dann das Gehäuse sofort mit Blut, was mit dem Sensor sicher und mit kurzer Ansprechzeit detektiert werden kann.

Das Gehäuse mit dem Sensor bildet bevorzugt eine kompakte Baueinheit, die ein kleines Bauvolumen aufweist und kostengünstig herstellbar ist. Bevorzugt ist als weiterer Bestandteil der Sicherungseinrichtung eine Auswerteeinheit zur Auswertung der vom Sensor generierten Sensorsignale vorgesehen. Die Auswerteeinheit befindet sich vorteilhaft außerhalb des Gehäuses, so dass deren Funktion nicht durch in das Gehäuse einströmendes Blut beeinträchtigt wird. Mit der Auswerteeinheit wird vorteilhaft auch das Alarmsignal generiert. Dieses kann im einfachsten Fall als optisches und/oder akustisches Signal ausgebildet sein. Alternativ oder zusätzlich kann das Alarmsignal ein elektrisches Signal sein, das beispielsweise zu einer Krankenstation geführt wird, so dass sich dort aufhaltendes Krankenpersonal dem Patienten sofort zu Hilfe eilen kann.

Der Sensor der Sicherungseinrichtung arbeitet generell nach dem Prinzip eines Näherungsschalters der erfasst, ob das auf den Gefäßzugang gesetzte Gehäuse blutleer oder mit Blut gefüllt ist. Der Sensor kann dabei prinzipiell als mechanischer, insbesondere piezoelektrischer, kapazitiver oder induktiver Sensor ausgebildet sein.

Besonders vorteilhaft ist der Sensor ein optischer Sensor mit einem Lichtstrahlen emittierenden Sender und einem Lichtstrahlen empfangenden Empfänger. Der Begriff Licht umfasst dabei generell elektromagnetische Strahlung sowohl im sichtbaren Wellenlängenbereich als auch im unsichtbaren Wellenlängenbereich, insbesondere im Infrarotbereich.

Dabei ist der optische Sensor ein Lichttaster, welcher ein binäres Ausgangssignal generiert.

Mit dem so ausgebildeten Sensor kann ein Einfließen von Blut in das Gehäuse schnell und sicher erfasst werden, so dass mit diesem optischen Sensor ein Herauslösen der Kanüle aus dem Gefäßzugang zuverlässig und mit kurzer Ansprechzeit detektiert werden kann.

Weiterhin ist vorteilhaft, dass der optische Sensor nur wenige, kostengünstig herstellbare Komponenten aufweist. Besonders vorteilhaft sind dabei als Sender eine Leuchtdiode und als Empfänger ein Photowiderstand oder eine Photodiode vorgesehen.

Um aus dem Gefäßzugang austretendes Blut sehr frühzeitig erkennen zu können, sind der Sender und der Empfänger an der Innenseite des Deckels des Gehäuses so angeordnet, dass die vom Sender emittierten Lichtstrahlen in Richtung des Gefäßzugangs emittiert und von dort zum Empfänger reflektiert werden. Alternativ kann mit dem optischen Sensor auch eine Absorptionsmessung durchgeführt werden.

Die Nachweisempfindlichkeit des optischen Sensors kann dadurch noch weiter erhöht werden, dass die Empfangssignale des Empfängers bei korrekt in den Gefäßzugang eingeführter Kanüle in einem Einlernvorgang als Referenzwerte in einer Auswerteeinheit gespeichert werden. Während einer auf den Einlernvorgang folgenden Betriebsphase werden die aktuellen Empfangssignale mit den Referenzwerten verglichen. Bei einem Herauslösen der Kanüle aus dem Gefäßzugang füllt dieses ausfließende Blut das Gehäuse zumindest partiell. Die Lichtstrahlen werden nur noch von dem einfließenden Blut reflektiert. Infolge der dadurch entstehenden Abweichung der aktuellen Empfangssignale wird von den Referenzwerten in der Auswerteinheit das Alarmsignal generiert.

Die erfindungsgemäße Sicherungseinrichtung kann prinzipiell im Bereich der Tiermedizin eingesetzt werden, besonders vorteilhaft jedoch im Bereich der Humanmedizin.

Ein wichtiges Einsatzgebiet stellt bei der Durchführung der Dialyse die Sicherung einer in die Vene eines Patienten eingeführten Kanüle dar. Bei der Durchführung der Dialyse, das heißt der Reinigung des Bluts des Patienten mittels einer Dialysemaschine, wird dem Patienten über eine in eine Arterie eingeführte Kanüle Blut entnommen, welches ihm von der Dialysemaschine aus über eine in eine Vene eingeführte Kanüle wieder zugeführt wird. Eine Unterbrechung dieser Zufuhr würde einen plötzlichen, großen Blutverlust für den Patienten bedeuten, der schnell lebensgefährlich wird. Derartige Gefahrensituationen können mit der erfindungsgemäßen Sicherungseinrichtung sicher vermieden werden.

Die erfindungsgemäße Sicherungseinrichtung kann vorteilhaft in weiteren medizinischen Anwendungen eingesetzt werden, wo das Herauslösen einer Kanüle zur Führung von Blut oder anderen Flüssigkeiten aus dem Gefäßzugang für den Patienten eine erhebliche Gefährdung bedeuten würde. Hierzu gehören Einsätze auf Intensivstationen von Krankenhäusern, um einem Patienten einen Katheder zu setzen oder eine Langzeitinfusion zu geben. Auch für den Fall, dass Kanülen mit Medikamenten führenden Schläuchen am Patienten festgenäht werden, ist die erfindungsgemäße Sicherungseinrichtung vorteilhaft einsetzbar. Schließlich sind Einsätze bei Liquor-Drainagen, wo ein Absaugen von Flüssigkeit aus dem Hirn oder zentralen Nervensystem eines Patienten erfolgt, möglich.

Die Erfindung wird im Folgenden anhand der Zeichnungen erläutert. Es zeigen:
- Figur 1:: Schematische Darstellung der erfindungsgemäßen Sicherungseinrichtung bei in einen Gefäßzugang eines Patienten eingeführter Kanüle.
- Figur 2:: Anordnung gemäß Figur 1 bei aus dem Gefäßzugang herausgelöster Kanüle.

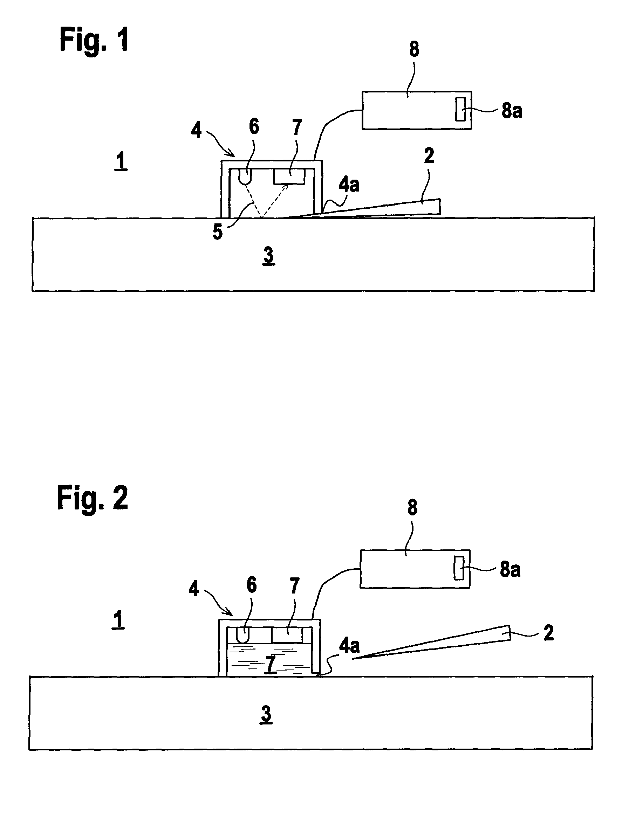

Die Figuren 1 und 2 zeigen schematisch ein Ausführungsbeispiel einer Sicherungseinrichtung 1, mittels derer kontrolliert wird, ob eine einen Injektionskörper bildende Kanüle 2 in einen Gefäßzugang eines Patienten ordnungsgemäß eingeführt ist oder ob diese aus dem Gefäßzugang herausgelöst ist. Bei dem Gefäßzugang kann es sich insbesondere um eine Einstichstelle handeln, wobei ohne Beschränkung der Allgemeinheit auf eine solche Bezug genommen wird. Generell sind jedoch auch andere Gefäßzugänge wie z.B. chirurgische Schnitte möglich. Figur 1 zeigt dabei die in eine Einstichstelle in einem Arm 3 eines Patienten eingeführte Kanüle 2, während Figur 2 die aus der Einstichstelle herausgelöste Kanüle 2 zeigt. Anstelle einer Kanüle 2 kann als Injektionskörper generell auch ein Schlauch verwendet werden, der mittels eines Katheders in die Blutbahn eingeführt wird.

Im vorliegenden Fall dient die Sicherungseinrichtung 1 zu Überwachungszwecken bei einer am Patienten durchzuführenden Dialyse. Dabei ist an die Kanüle 2 ein nicht dargestellter Schlauch angeschlossen, über welchen gereinigtes Blut aus einer nicht dargestellten Dialysemaschine dem Patienten über die Kanüle 2 in eine Vene eingeführt und damit rückgeführt wird.

Die Sicherungseinrichtung 1 weist ein Gehäuse 4 mit einem darin angeordneten optischen Sensor auf. Das Gehäuse 4 besteht aus einer im Wesentlichen quaderförmigen Kunststoff-Kapsel, die an ihrer Unterseite offen ist. An einer Seitenwand des Gehäuses 4 kann eine kleine Aussparung 4a vorgesehen sein, über welche die Kanüle 2 in den Innenraum des Gehäuses 4 eingeführt wird. Die Größe des Gehäuses 4 ist auf die Dimension der Kanüle 2 abgestimmt, so dass, wie aus Figur 1 ersichtlich, bei in die Einstichstelle eingeführter Kanüle 2 der Bereich mit der Einstichstelle und der Kanülenspitze von dem Gehäuse 4 umschlossen wird, wenn es mit seiner offenen Unterseite auf den Arm 3 des Patienten aufgesetzt wird.

Der optische Sensor weist als Sensorkomponenten einen Lichtstrahlen 5 emittierenden Sender 6 und einen Lichtstrahlen 5 empfangenden Empfänger 7 auf, welche an der Innenseite des Deckels des Gehäuses 4 angeordnet sind. Der Sender 6 und der Empfänger 7 sind an eine Auswerteinheit 8 außerhalb des Gehäuses 4 angeschlossen. Der Sender 6 ist von einer Leuchtdiode gebildet, der Empfänger 7 besteht aus einem Photowiderstand oder einer Photodiode. Die Auswerteeinheit 8 besteht aus einem Microcontroller oder dergleichen. Der optische Sensor bildet einen Lichttaster. Dessen Sensorkomponenten sind so dimensioniert, dass bei ordnungsgemäß in die Einstichstelle eingeführter Kanüle 2 die Lichtstahlen 5 des Senders 6 oder ein großer Teil hiervon, von der Einstichstelle, das heißt vom Arm 3 des Patienten und von der Kanüle 2 zurück zum Empfänger 7 reflektiert werden.

Im Alarmfall, wenn sich wie in Figur 2 dargestellt die Kanüle 2 selbsttätig aus der Einstichstelle löst, füllt sich der vom Arm 3 und dem Gehäuse 4 umschlossene Hohlraum schnell mit Blut, so dass die vom Sender 6 emittierten Lichtstrahlen 5 durch das Blut stark abgedämpft werden und nicht mehr oder nur noch in geringem Umfang zum Empfänger 7 gelangen. Dabei zeigt Figur 2 den Fall einer nahezu vollständigen Füllung F des Gehäuses mit Blut.

Diese beiden Fälle werden im optischen Sensor dadurch unterschieden, dass in diesem ein binäres Ausgangssignal mit zwei Schaltzuständen generiert wird. Der erste Schaltzustand entspricht dem fehlerfreien Zustand bei ordnungsgemäß in die Einstichstelle eingeführter Kanüle 2, der zweite Schaltzustand entspricht einem Alarmsignal, dass meldet wenn Blut aus der Einstichstelle bei dort herausgelöster Kanüle 2 austritt.

Die Zuordnung der Empfangssignale des Empfängers 7 zu einem der Schaltzustände erfolgt zweckmäßig über einen Referenzwertvergleich. In einem Einlernvorgang werden die Empfangssignale in der Auswerteeinheit 8 eingelernt, die bei fehlerfreiem Fall, das heißt bei in die Einstichstelle eingeführter Kanüle 2, erhalten werden. Diese Werte werden in der Auswerteeinheit 8 als Referenzwerte eingelernt. In der auf den Einlernvorgang folgenden Betriebsphase werden die aktuell ermittelten Empfangssignale mit den Referenzwerten verglichen. Stimmen die aktuellen Empfangssignale mit den Referenzwerten innerhalb vorgegebener Toleranzgrenzen überein, wird das in der Auswerteeinheit 8 als fehlerfreier Zustand gewertet, so dass dann das Ausgangssignal den ersten Schaltzustand einnimmt. Liegen dagegen die Empfangssignale außerhalb der Toleranzen der Referenzwerte, nimmt das Ausgangssignal den zweiten Schaltzustand ein.

Das Ausgangssignal des Sensors wird über einen Ausgang 8a der Auswerteinheit 8 ausgegeben, beispielsweise um einen optischen oder akustischen Alarmgeber anzusteuern. Wird über den Ausgang 8a das erste Schaltsignal ausgegeben, bleibt der Alarmgeber deaktiviert. Wird dagegen der zweite Schaltzustand, das heißt das Alarmsignal, über den Ausgang 8a ausgegeben, wird der Alarmgeber aktiviert und gibt eine optische oder akustische Alarmmeldung aus. Damit wird das Krankenpersonal alarmiert und kann dem Patienten sofort Hilfe eilen.

### Bezugszeichenliste

- (1): Sicherungseinrichtung
- (2): Kanüle
- (3): Arm
- (4): Gehäuse
- (4a): Aussparung
- (5): Lichtstrahlen
- (6): Sender
- (7): Empfänger
- (8): Auswerteinheit
- (8a): Ausgang
- (F): Füllung

## Patentansprüche

1. Sicherungseinrichtung (1) zur Kontrolle eines Gefäßzugangs eines Lebewesens, **gekennzeichnet durch** einen Sensor, welcher in oder an einem den Gefäßzugang umgebenden Gehäuse (4) angeordnet ist, wobei mit dem Sensor eine in das Gehäuse (4) von dem Gefäßzugang aus einfließende Flüssigkeit, insbesondere Blut, detektiert wird.

2. Sicherungseinrichtung nach Anspruch 1, **dadurch gekennzeichnet, dass** mit dieser ein Alarmsignal generiert wird, falls mittels des Sensors ein Einfließen der Flüssigkeit in das Gehäuse (4) detektiert wird.

3. Sicherungseinrichtung nach einem der Ansprüche 1 oder 2, **dadurch gekennzeichnet, dass** mit dem Sensor ein Herauslösen einer Kanüle (2) oder eines Katheders aus dem Gefäßzugang erfasst wird.

4. Sicherungseinrichtung nach einem der Ansprüche 1 bis 3, **dadurch gekennzeichnet, dass** das Gehäuse (4) an seiner Unterseite eine Öffnung aufweist, welche auf die Haut des Lebewesens aufsetzbar ist, wobei durch das Aufsetzten ein von der Haut und dem Gehäuse (4) im Wesentlichen geschlossener Hohlraum entsteht, in welchem die Kanüle (2) oder der Katheder an den Gefäßzugang geführt ist.

5. Sicherungseinrichtung nach Anspruch 4, **dadurch gekennzeichnet, dass** eine Wand des Gehäuses (4) eine Aussparung (4a) zur Einführung der Kanüle (2) oder des Katheders aufweist.

6. Sicherungseinrichtung nach einem der Ansprüche 1 bis 5, **dadurch gekennzeichnet, dass** der Sensor ein optischer Sensor mit einem Lichtstrahlen (5) emittierenden Sender (6) und einem Lichtstrahlen (5) empfangenden Empfänger (7) ist.

7. Sicherungseinrichtung nach Anspruch 6, **dadurch gekennzeichnet, dass** der optischer Sensor ein Lichttaster ist, welcher ein binäres Ausgangssignal generiert.

8. Sicherungseinrichtung nach einem der Ansprüche 6 oder 7, **dadurch gekennzeichnet, dass** der Sender (6) und der Empfänger (7) an der Innenseite des Deckels des Gehäuses (4) so angeordnet sind, dass die vom Sender (6) emittierten Lichtstrahlen (5) in Richtung des Gefäßzugangs emittiert werden und von dort zum Empfänger (7) reflektiert werden.

9. Sicherungseinrichtung nach Anspruch 8, **dadurch gekennzeichnet, dass** die Empfangssignale des Empfängers (7) bei korrekt in den Gefäßzugang eingeführter Kanüle (2) oder korrekt eingeführtem Katheder in einem Einlernvorgang als Referenzwerte in einer Auswerteeinheit (8) gespeichert werden, und dass während einer auf den Einlernvorgang folgenden Betriebsphase die aktuellen Empfangssignale mit den Referenzwerten verglichen werden.

10. Sicherungseinrichtung nach Anspruch 9, **dadurch gekennzeichnet, dass** bei einem Herauslösen der Kanüle (2) oder des Katheders aus dem Gefäßzugang über diese ausfließende Flüssigkeit das Gehäuse (4) zumindest partiell füllt, so dass die Lichtstrahlen (5) nur noch von der einfließenden Flüssigkeit reflektiert werden, und dass infolge der dadurch entstehenden Abweichung der aktuellen Empfangssignale von den Referenzwerten in der Auswerteinheit (8) das Alarmsignal generiert wird.

11. Sicherungseinrichtung nach Anspruch 9, **dadurch gekennzeichnet, dass** mit dem optischen Sensor eine Absorptionsmessung durchgeführt wird.

12. Sicherungseinrichtung nach einem der Ansprüche 9 bis 11, **dadurch gekennzeichnet, dass** die Auswerteeinheit (8) außerhalb des Gehäuses (4) angeordnet ist.
